# EUROPEAN PATENT APPLICATION

(11) **EP 3 871 661 A1**
(43) Date of publication of application: **01.09.2021**
(21) Application number: 20753264.9
(22) Date of filing: 06.02.2020
(51) Int. Cl.: A61K 9/00, A61K 9/19, A61K 47/28, A61K 47/26, A61K 31/661, A61P 3/04

(54) **TOPICAL INJECTABLE COMPOSITION**

(30) Priority: 08.02.2019 KR 20190015232
(71) Applicant: Jetema Co., Ltd, Gangwon-do 26355 (KR)
(72) Inventor: KIM, Jae Young, Seoul 05649 (KR); NAM, Jeong Sun, Seoul 06076 (KR)
(74) Representative: Louis Pöhlau Lohrentz
(86) International application number: PCT/KR2020/001686
(87) International publication number: WO 2020/162685

(57) **Abstract**

The present invention relates to a dry powder formulation for injection of a DCA ingredient. The present invention is a dry powder formulation, which has excellent storage stability, has a pH of 8.2 or less so that, when dissolved in injection water, pain is lessened during injection by forming a pH environment closer to that of the human body than conventional injections, and allows DCA to dissolve well in injection water without precipitating.

## Description

### TECHNICAL FIELD

### CROSS-REFERENCE TO RELATED APPLICATIONS

This application claims the benefit of priority based on Korean Patent Application No. 10-2019-0015232 filed on February 8, 2019, and all contents disclosed in the documents of the Korean patent application are incorporated as part of this specification.

The present invention relates to an injectable composition that includes deoxycholic acid. Specifically, it relates to a composition for dry powder formulation used in injections excellent for non-surgically removing fat from a person with locally deposited fat while minimizing pain, swelling or other side effects.

### BACKGROUND ART

### [Fat Removal Procedure]

Locally deposited fat on the body like the face is not good from a cosmetic point of view. For example, with a double chin or deep cheeks, the face may look bigger.

Such localized fat deposits occur according to aging, lifestyle, or genetic factors. To overcome this, there are various exercise regimens and diets but with limited effectiveness . Thus, it has led to the advent of surgical therapy for local fat reduction.

The widely used surgical therapies include liposuction, lipoplasty, liposuction incision, and so forth. However, the surgical therapies have a limitation that they take several weeks to heal, a longer healing period for some patients, such as diabetics, and carry the risk of side effects, including excessive bleeding, internal organ damage, bacterial infection, scarring, or pain.

### [PPC Injection for Fat Reduction]

Therefore, injections for local fat reduction have emerged as an alternative to surgical therapy. They consist of ingredients that decompose fat cells, and are used by injecting drugs into the subcutaneous fat layer. Lipostavil, a mixture of PPC and DCA, is a representative example of local fat reducing injection.

PPC is an essential phospholipid, a major component of cell membranes. In 1988, Dr. Sergio Magiori from Italy reported the PPC usage for the treatment of blepharoglobinoma, a disease involved in yellow fat deposits on the eyelids . In the mid-1990's, Dr. Patricia Rittes published the use of PPC for removing infraorbital fat tissue. This initiated widespread public interest in PPC usage for lipolysis treatment.

On the other hand, when used as an injection, the ingredients need to be dispersed in a small particle size. If ingredients prone to precipitation rather than solubilization are injected, large particles possibly block blood vessels, adversely affecting the blood flow of tissues around the blocked blood vessels or damaging or irritating the tissue to cause itching and pain. Yet, PPC is a waxy solid substance that is insoluble in water for injection.

For this reason, DCA is used as a solubilizing agent to disperse PPC to a particle size of 10 nm or less. DCA is one of the bile acids.

### [DCA Injection for Fat Reduction]

In the off-label treatment of Lipostavil, a mixture of PPC and DCA, for local fact reduction, there are side effects, such as pain, local edema, erythema, hardening, paresthesia, itchiness, and burning sensation.

Korean Patent Publication No. 1217497 discloses a DCA sodium salt injection with the PPC content reduced or eliminated, claiming that PPC causes the above side effects and further that local fats can be emulsified solely with bile salts.

In April 2015, Kisera was approved as an injection for double chin reduction by the U.S. Food and Drug Administration. Approved as a specialized medication to improve the appearance and profile with a nonsurgical injection technique, Kybella is greatly expected in the facial plastic industry, following Botox, Natrelle, and Juvederm.

### [Prior Technical Documentation]

### [Patent Documents]

Patent Document 1: Korean Registered Patent Publication No. 1217497
Patent Document 2: Korean Registered Patent Publication No. 1751585

### DISCLOSURE OF INVENTION

### TECHNICAL PROBLEM

Kybella, a DCA-based fat-removing injection, is in an alkaline environment with a pH value over 8.2. Korean Registered Patent Publication No. 1751585 attributes the alkaline environment of Kybella to the precipitation of DCA. In other words, according to the cited patent, DCA forms a precipitate in no more than four weeks in the pH range from 7.56 to 8.09, so it is unavailable as an injection for long-term distribution and stable only at pH 8.21-8.55, producing no precipitate.

It is a common sense that the pH and osmotic pressure of injections different from those of the human body cause pain. In other words, the pH of the human body is typically about 7.4-7.5, so pain is unavoidable when the pH of the injection is above 8.21. Despite this fact, however, the pH of Kybella is inevitably adjusted to the range causing pain due to the precipitation of DCA.

The inventors of the present invention have attempted to develop a composition for DCA injection having a pH value approximating to the pH of the human body in order to reduce injection pain. It is therefore an object of the present invention to develop an injectable composition that contains DCA sufficiently solubilized without precipitation when injected, has a pH value approximating to the pH of the human body, causing less pain, and exhibits availability for long-term distribution.

### TECHNICAL SOLUTION

The present invention can solve the above-described problems by the following means. A feature of the present invention is that DCA or its salt is implemented into a dry powder formulation, which is a novel formulation that has never been marketed.
(1) A dry powder formulation for injection containing DCA or its salt.
(2) The dry powder formulation for injection according to (1), wherein the pH is 8.2 or less.
(3) The dry powder formulation for injection according to (1) or (2), wherein the pH is 8.0 or less.
(4) The dry powder formulation for injection according to any one of (1), (2) and (3), wherein the pH is 7.4-7.9.
(5) The dry powder formulation for injection according to any one of (1) to (4), wherein the dry powder formulation includes sugar or sugar alcohol as an excipient.
(6) The dry powder formulation for injection according to (5), wherein the sugar or sugar alcohol is mannitol.
(7) The dry powder formulation for injection according to any one of (1) to (6), wherein the dry powder formulation for injection is not required to contain a preservative.
(8) A use of the dry powder formulation for injection according to any one of (1) to (7) in the manufacture of a formulation for induction of lipolysis or treatment of an obesity disease.
(9) A use of the dry powder formulation for injection according to any one of (1) to (7) for induction of lipolysis or treatment of an obesity disease.
(10) A method for inducing lipolysis or preventing or treating an obesity disease that includes administering a therapeutically effective amount of the dry powder formulation for injection according to any one of (1) to (7) to a mammal, including a human being.

In the method according to any one of (8), (9) and (10), the obesity may include, but not limited to, local obesity.

In the method according to (8), the dry powder formulation for injection according to the present invention for the manufacture of a formulation for treating an obesity disease may include a pharmaceutically acceptable carrier or the like, and may further include other agents.

In the method according to (10), the term "therapeutically effective amount" refers to an amount of a drug effective for the treatment of an obesity disease, e.g., an amount of a dry powder formulation for injection administered to a biological individual subject, including any amount of the dry powder formulation for injection to make an effect of preventing occurrence or recurrence of an obesity disease, alleviating symptoms, inhibiting direct or indirect pathological consequences, preventing metastasis, lowering the rate of progression, alleviating or temporarily reducing the condition, or improving the prognosis. In other words, the therapeutically effective amount may be interpreted as encompassing all doses by which the dry powder formulation for injection can improve or cure the symptoms of the obesity disease.

The method for preventing or treating an obesity disease includes administering a dry powder formulation for injection not only to deal with the disease itself prior to the onset of the symptoms of the disease, but also to inhibit or avoid the symptoms of the disease. In the management of a disease, the prophylactic or therapeutic dose of a specific active ingredient varies depending on the nature and severity of the disease or condition and the route of administration of the active ingredient. The dose and frequency also vary depending on the age, weight and response of the individual patient. A suitable dosage regimen can be readily selected by those skilled in the art taking these factors into account. In addition, the treatment method of the present invention may further include administering a therapeutically effective amount of an additional active agent used to the treatment of an obesity disease together with the dry powder formulation for injection. The additional active agent may exhibit a synergistic or additive effect with the dry powder formulation for injection.

The mammal including a human being may include mammals, such as humans, monkeys, cows, horses, dogs, cats, rabbits, and rats.

### EFFECTS OF INVETNION

The present invention is stable for at least 30 months from the date of manufacture and excellent in storage stability even for a longer period of time. As the present invention has a pH value of 8.2 or less when dissolved in water for injection, it causes less pain than the conventional formulations and is clearly dissolved in the water for injection without causing precipitation of DCA.

In addition, the present invention can be easily dissolved in water for injection at high dissolution rate without any special stirring mechanism, causing little inconvenience in use.

In addition, the present invention has a manufacturing advantage such as good formation of a cake when making a dry powder formulation.

In addition, even if not containing a preservative particularly harmful to the human body, the present invention has no problem in terms of acceptance criteria for biological contamination or the like.

### BRIEF DESCRIPTION OF DRAWINGS;

FIG. 1 presents the dissolution of a DCA ingredient itself (left) and the DCA dry powder formulation (right) of the present invention in water for injection at pH 7.0, where the images from top to bottom show before dissolution, 10 seconds after dissolution, and 30 seconds after dissolution, respectively.
FIG. 2 presents the dissolution of a DCA ingredient itself (left) and the DCA dry powder formulation (right) of the present invention in water for injection at pH 7.8, where the images from top to bottom show before dissolution, 10 seconds after dissolution, and 30 seconds after dissolution, respectively.
FIG. 3 presents images showing the appearance of DCA dry powder formulations according to the embodiments of the present invention, i.e., Examples 1, 2 and 3 from left to right.
FIG. 4 presents the dissolution of the DCA dry powder formulation of the present invention in water for injection, where the images from top to bottom show before dissolution, 10 seconds after dissolution, 30 seconds after dissolution, and 8 hours after dissolution, respectively.

### BEST MODES FOR CARRYING OUT THE INVENTION

The dry powder formulation has to be dissolved in water for injection before used as an injection. The water for injection may be general neutral sterile water for injection. The typical pH of the water for injection commercially available may range from about 7.0 to about 7.8.

In this regard, the amount of the dry powder formulation may be the same as that of the conventional product, Kybella. For example, the active ingredient may be formulated at a concentration of 0.4% w/v to 2% w/v, which is reported to be effective in eliminating a buildup of fat in the body. Preferably, the active ingredient may be formulated at a concentration of 0.5% w/v to 1% w/v.

Hereinafter, the present invention will be described in further detail with reference to the following examples, which are not construed to limit the scope of the present invention.

### [Examples]

A dry powder formulation was manufactured according to the composition given in Table 1 below (Example 1).

**[Table 1]**

| Ingredients | Contents |
|---|---|
| Deoxycholic acid | 50.0 mg |
| D-mannitol | 273.7 mg |
| Sodium hydroxide | q.s. |
| Hydrochloric acid | q.s. |

1. After dissolving D-mannitol in 70% of the final volume of the water for injection, add deoxycholic acid into a suspension.
2. Gradually add 10 M sodium hydroxide until the solution becomes clear (at least pH 10).
3. To the completely dissolved deoxycholic acid solution, gradually add 1M hydrochloric acid to adjust the pH value to 7.8.
4. Mark the final volume (5 mL).
5. Dry the powder product, where Example 1 has a pH value of 7.8.

### [Precipitation Test]

The DCA ingredient itself (Comparative Example) and the dry powder formulation (Example 1) as a novel DCA formulation manufactured according to the present invention were dissolved in water for injection at pH 7.0 and 7.8. The solution was agitated simply by hand without a separate device in the same way that a dry powder formulation is generally dissolved in water for injection prior to administration by injection in a clinic.

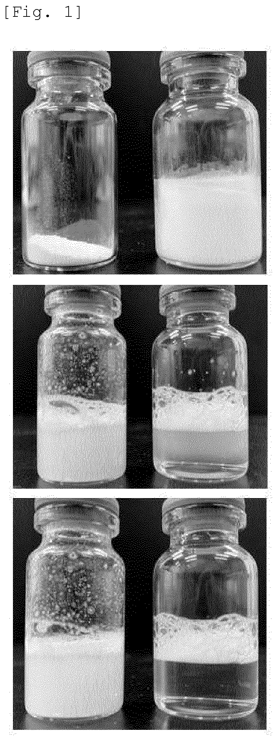

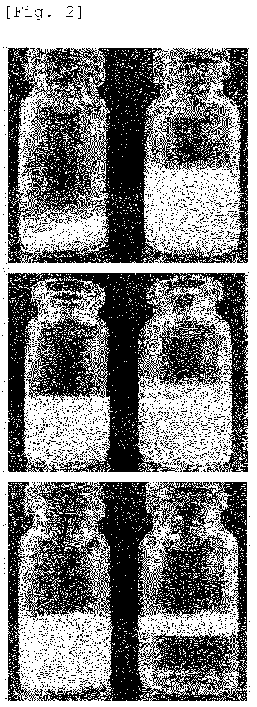

As a result, as shown in FIGS. 1 and 2, the DCA ingredient itself did not dissolve easily but formed a precipitate, while the DCA dry powder formulation dissolved sufficiently in 30 seconds and formed no precipitate. Despite the similar pH environment, the DCA ingredient itself and the dry powder formulation of the present invention were noticeably different in the degree of dissolution from each other.

FIG. 1 shows the dissolution in water for injection at pH 7.0, and FIG. 2 shows the dissolution in water for injection at pH 7.8. In FIGS. 1 and 2, the images from top to bottom show before dissolution, 10 seconds after dissolution, and 30 seconds after dissolution, respectively.

Particularly, the DCA dry powder formulation had a pH value of about 7.8, so even if it was dissolved to reconstitute in the water for injection at pH 7.0 and pH 7.8, the pH value was maintained at 8.2 or below. Unlike the conventional perception, the DCA dry powder formulation was rapidly dissolved without precipitation of DCA.

### [Stability Test]

The dry powder formulation (Example 1) as a novel DCA formulation manufactured according to the present invention was evaluated in regards to the stability under the conditions as follows.

**[Table 2]**

| Items | Conditions |
|---|---|
| Storage container | Vial: Borosilicate glass |
| | Rubber stopper: Chlorobutyl, medical |
| Storage conditions | Long-term storage conditions: 25±2°C, 60+5% |
| | Accelerated conditions: 40±2°C, |
| | 75+5% |
| Storage period | 3 months |

As a result, the Example 1 according to the present invention as follows had neither generation of related substances nor pH variations even under the long-term storage conditions and accelerated conditions. In addition, it did not have any change in appearance occasionally occurring in some solid formulations.

**[Table 3]**

| Items | Initial | After 3 months under long-term storage conditions (25±2°C, 60±5%) | After 3 months under accelerated conditions (40±2°C, 75±5%) |
|---|---|---|---|
| Appearance | Suitable | Suitable | Suitable |
| Content | 96.7% | 96.7% | 97.3% |
| Related substance | N/D | N/D | N/D |
| pH | 7.8 | 7.8 | 7.8 |

### [Dissolution Rate and Manufacturability]

A comparison was made in regards to the appearance properties after reconstitution and manufacture as a function of the type of the excipient.

A dry powder formulation was manufactured under the same conditions of Example 1, excepting that the excipient was replaced by lactose (Example 2) or fructose (Example 3).

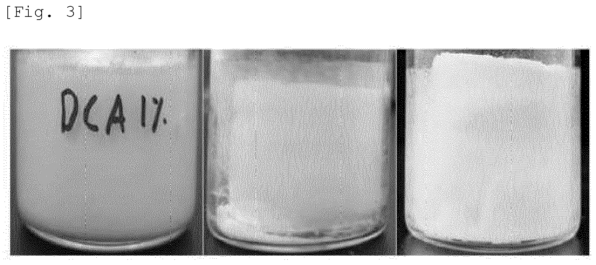

As a result, both the dry powder formulations were manufactured so that the appearance properties were more stable particularly when using mannitol in Example 1 (Refer to FIG. 3) . In other words, as can be seen from FIG. 3 in which the images from left to right show the cases of using mannitol (Example 1), lactose (Example 2) and fructose (Example 3), respectively, all the examples had a cake formation without any issue, but the Example 1 using mannitol was the best in the degree of cake formation, suggesting that the use of mannitol resulted in the highest manufacturability.

The rate of dissolution upon reconstitution and the precipitation after dissolution were determined.

Water for injection was added to each of the Examples 1, 2 and 3, and after agitation for 5 seconds, the Examples 1, 2 and 3 were measured in regards to the degree of dissolution over time and stood at the room temperature for 8 hours to determine whether a precipitate was formed or not.

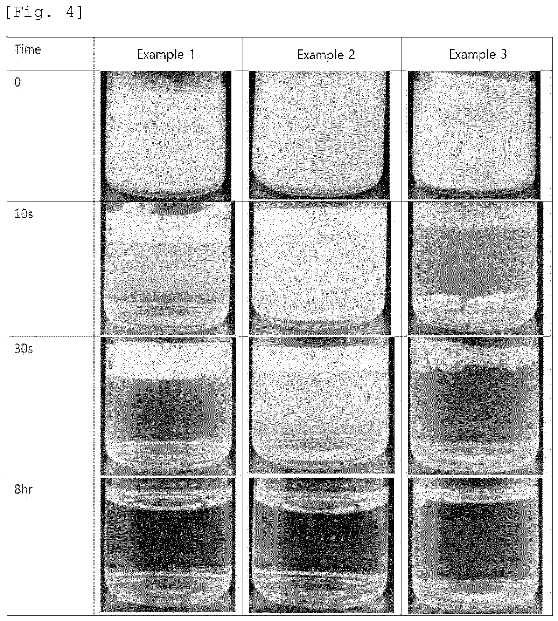

As a result, all of the Example 1, 2 and 3 were completely dissolved in 30 seconds. Particularly, the Example 1 using mannitol was most quickly and completely dissolved. Further, none of the Examples 1, 2 and 3 formed a precipitate over 8 hours of dissolution (Refer to FIG. 4).

### Industrial Availability

The present invention is directed to a dry powder formulation for injection that includes DCA or its salt. Specifically, it relates to a composition of a dry powder formulation used in injections excellent for non-surgically removing fat from a person with locally deposited fat while minimizing pain, swelling, or other side effects.

The dry powder formulation for injection containing DCA or its salt according to the present invention is stable for at least 30 months from the date of manufacture and excellent in storage stability even for a longer period of time. As the present invention has a pH value of 8.2 or less when dissolved in water for injection, it causes less pain than the conventional formulations and is clearly dissolved in the water for injection without causing precipitation of DCA.

## Claims

1. A dry powder formulation for injection comprising DCA or a salt thereof.

2. The dry powder formulation for injection according to claim 1, wherein the dry powder formulation for injection has a pH value of 8.2 or less.

3. The dry powder formulation for injection according to claim 1, wherein the dry powder formulation for injection comprises sugar or sugar alcohol as an excipient.

4. The dry powder formulation for injection according to claim 1, wherein the dry powder formulation for injection is not required to comprise a preservative.

5. A use of the dry powder formulation for injection according to any one of claims 1 to 4 in inducing lipolysis or manufacturing a formulation for obesity disease.

6. A use of the dry powder formulation for injection according to any one of claims 1 to 4 for inducing lipolysis or treating an obesity disease.

7. A method for inducing lipolysis or preventing or treating an obesity disease, the method comprising administering a therapeutically effective amount of the dry powder formulation for injection according to any one of claims 1 to 4 to a mammal including a human being.
